# EUROPEAN PATENT SPECIFICATION

(11) **EP 3 184 082 B1**
(45) Date of publication and mention of the grant of the patent: **17.08.2022**
(21) Application number: 15202542.5
(22) Date of filing: 23.12.2015
(51) Int. Cl.: A61F 2/24

(54) **STENT FOR A SURGICAL VALVE**
STENT FÜR EIN CHIRURGISCHES VENTIL
STENT DESTINÉ À UNE VALVE CHIRURGICALE

(43) Date of publication of application: 28.06.2017
(73) Proprietor: P+F Products + Features Vertriebs GmbH, 1190 Wien (AT)
(72) Inventor: Kiss, Katharina, 1190 Wien (AT)
(74) Representative: Manitz Finsterwald Patent- und Rechtsanwaltspartnerschaft mbB

(56) References cited:
- WO-A1-2007/054015
- US-A1- 2010 049 313
- US-A1- 2014 249 622
- US-A1- 2014 277 412
- US-A1- 2014 343 670

## Description

The present invention relates to a medical implant or prosthesis, such as a stent for a surgical valve, for the treatment of tricuspid valve leakage or regurgitation and of mitral valve leakage or regurgitation. There is also described a method, none of the surgical methods herein described forming part of the invention, of delivering and deploying the medical implant or prosthesis.

Healthy heart function provides necessary blood flow to the body. The anatomy of the heart is comprised of four chambers: the right atrium, right ventricle, left atrium and left ventricle. There are valves located between the right chambers (tricuspid valve) and the left chambers (mitral valve). Blood travels from the caval veins, the superior vena cava and inferior vena cava, to the right atrium. From the right atrium, blood passes through the tricuspid valve into the right ventricle. From there, blood flows through the pulmonary valve to the lungs. Following oxygenation, blood flows back to the left atrium, through the mitral valve and into the right ventricle. Blood is then pushed through the aortic valve and from there, ultimately, to the rest of the body.

The tricuspid valve is located between the right atrium and ventricle, whereas the mitral valve is located between the left atrium and ventricle. The native valves consist of two and three leaflets respectively for the mitral and the tricuspid valve. The base of these leaflets is attached to the opening connecting both the atrium and the ventricle, creating a non-circular valve annulus. Chordae tendinae are attached to the caudal ends of the leaflets, connecting the leaflets to the myocardial wall via papillary muscles.

Proper opening and closing of the atrioventricular valves is dependent on the function of all of the structures involved with leaflet function, specifically the annulus, the leaflets, the chordae tendinae, the papillary muscles and a healthy myocardial wall. During ventricular filling (diastole), the atrioventricular valves remain open and during ventricular contraction (systole), the valves close as a consequence of complete leaflet apposition. In instances where complete apposition of the leaflets is not achieved, leakage or regurgitation across the valve affects cardiac performance and health. Severe regurgitation or leakage can lead to hemodynamic deterioration and/or heart failure.

Moderate or higher tricuspid valve regurgitation affects cardiac functional performance, and is typically indicative of a broader primary disease such as left heart dysfunction. Primary dysfunction can include pulmonary hypertension, right ventricular volume overload and right ventricular disease. Similarly, mitral regurgitation or leakage is primarily due to degenerative valve disease. The presence of dysfunctional support structures also contributes to mitral regurgitation, specifically as a consequence of a heart attack (ischemic mitral regurgitation) or ventricular dilation (functional mitral regurgitation). Other causes of atrioventricular regurgitation include acquired conditions (i.e. endocarditis, rheumatic heart disease) and congenital anomalies.

Atrioventricular regurgitation also impact the caval veins leading to the atrium. During tricuspid regurgitation, pressure and flow changes in the right atrium causes the inferior vena cava (IVC) and the superior vena cava (SVC) to dilate. Additionally, veins feeding the IVC and the SVC are also impacted by these effects. Specifically, tricuspid regurgitation causes the azygos and the hepatic veins, which are located cranial and caudal to the right atrial opening respectively, to dilate. This leads to overall hemodynamic deterioration and liver dysfunction. Physical symptoms of tricuspid regurgitation include fatigue, loss of appetite and abdominal fullness. Similarly, mitral regurgitation impacts the pulmonary vein and can cause fluid buildup in the lungs. Chronic mitral regurgitation often leads to left ventriclar re-modeling or dilation of the left ventricle. Physical symptoms of mitral regurgitation include fatigue, decreased exercise tolerance, shortness of breath, and swollen feet. If left untreated, either atrioventricular severe regurgitation can lead to pulmonary hypertension, atrial fibrillation or heart failure.

Prior art stents that can be used for the heterotopic treatment of atrioventricular regurgitation are disclosed in US2010/049313 A1, WO2007/054015 A1, and US2014/249622. In particular US2010/049313 discloses a prosthetic heart valve and a heart valve delivery apparatus for delivery of the prosthetic heart valve to a native valve site via the human vasculature. The valve can be self-expanding and comprises an expandable stent that is shaped to maintain the valve in the aortic annulus against axial without anchors or retaining devices that engage the surrounding tissue

For this reason it is an object of the present invention to provide a medical implant or prosthesis that can provide relief from tricuspid valve leakage or regurgitation and from mitral valve leakage or regurgitation. Moreover, it is a further object of the present invention to minimize the recovery time of a patient following the treatment with such a medical implant or prosthesis.

This object is satisfied by a stent for a surgical valve having the features of claim

A stent for a surgical valve for the heterotopic treatment of atrioventricular regurgitation in accordance with the invention is expandable from an un-deployed state into a deployed state, and comprises a frame composed of a plurality of first arms forming three interconnected sections, a proximal section, a distal section and a middle section disposed between the proximal section and the distal section. In the deployed state, a maximum outer diameter of the middle section is larger than a maximum outer diameter of the proximal section and a length of the proximal section corresponds to 35 to 70% of a length of the middle section, wherein the outer diameter of the middle section increases at a transition from the proximal section to the middle section. The proximal section has an at least substantially equal outer diameter over its length. At a transition between the proximal section and the middle section, the outer diameter of the middle section increases starting from the proximal section to the maximum outer diameter of the middle section, wherein, starting from the maximum outer diameter the outer diameter of the middle section decreases towards a transition located between the middle section and the distal section and Moreover, the plurality of first arms is outwardly directed in the distal section of the frame in the deployed state.

Thus, the present disclosure relates to the invention of a medical device for a minimally invasive preferably heterotopic treatment of tricuspid valve leakage or regurgitation and other similar cardiac conditions. Heterotopic placement indicates that the prosthesis will not be implanted directly at the annulus of the tricuspid valve. To treat tricuspid valve regurgitation, implants should be placed at the atrial junction of the Superior Vena Cava (SVC) and/or of the Inferior Vena Cava (IVC) - termed caval veins.

Due to the possible placement of such a surgical valve comprising such a stent at a heterotopic location, it is reasonable to expect that the presence of valves at the atriocaval junction may impact backflow into the veins. Impacting the backflow into the veins can cause an increase in the pressure inside the right atrium and thus limit regurgitation across the tricuspid valve thereby enhancing the function of the heart once such a surgical valve has been deployed.

Furthermore, the provision of a middle section that has a larger outer diameter than the proximal section means that the middle section of the valve can be attached to a vein by means of an interference fit once the valve has been expanded into the deployed state.

The expansion is made possible by the plurality of first arms that are interconnected in such a way that following the expansion they adapt to the anatomical need of the location of the implant. In this way the design of such a stent is adapted to accommodate the anatomical needs and implantation locations. The stent may consist of an expandable (e.g. balloon or self) stent frame. Moreover, between 6 and 50 first arms can be provided to form the stent frame of a surgical valve.

Selecting the length of the middle section to be longer than that of the proximal section enables the interference fit between the surgical valve and either one of the SVC and the IVC to be sufficient to prevent the surgical valve from becoming dislodged in time.

Moreover, providing a comparatively long proximal section having an at least substantially equal outer diameter over its length or an outer diameter that converges only slightly towards the middle section generates a uniform space required for the provision of a valve that ensures the correct function of such an implant or prosthesis.

Preferably the interconnected sections are interconnected by means of the plurality of first arms that extend from the proximal section via the middle section to the distal section. The plurality of arms are connected to one another at specific points along the length of the respective arms to form a web or net like structure.

Further, through the use of the plurality of first arms and the fact that the implant can be expanded such implant prostheses have the ability to be deployed via less-invasive or surgical means and/or transcatheter techniques, this considerably reduces the rehabilitation time of a patient and hence helps the improvement of a patient's health.

To this end it should be noted that the plurality of first arms can be uniformly or non-uniformly distributed along the circumference of the frame in order to enhance specific properties of specific sections of the stent.

Further embodiments of the invention are described in the dependent claims.

Preferably the stent further comprises a plurality of second arms that outwardly extend to a maximum outer diameter of the distal section, with the plurality of second arms extending beyond a distal end of the first arms. These arms can then be used to additionally fix the surgical valve to the SVC or the IVC as the case may be.

By forming the plurality of second arms such that they extend beyond a distal end of the first arms, the second arms can take over an additional anchoring function.

It is preferred when the maximum outer diameter of the distal section is at least approximately the same as a maximum outer diameter of the middle section in the deployed state. In this way the interference fit of the stent can engage at two different positions of the SVC and the IVC, thereby enhancing the attachment between a surgical valve and the blood vessel.

The outwardly directed first arms and/or the outwardly directed second arms can advantageously be configured to act like barbs or hooks in the deployed state of the implant or prosthesis in order to ensure that a surgical valve remains at its intended location, the location at which it was deployed.

It is preferred if the stent comprises further anchoring features, such as barbs or hooks, that are provided on the frame. In this way a connection between the surgical valve and either one of the IVC or SVC can be improved.

Advantageously a transition between the middle section and the distal section has an outer diameter that is less than the maximum outer diameter of the middle section and less than an outer diameter of the distal section in the deployed state. The provision of such a transition between the middle section and the distal section provides the frame with stability to increase its lifetime and thereby increase the period between separate surgeries that may be necessary to replace the valve in time. This reduces pain and the like for the patient.

Preferably, a diameter of the transition at least substantially corresponds to the maximum outer diameter of the proximal section or is less than the maximum outer diameter of the proximal section in the deployed state. Selecting the transition to have essentially the same diameter as that of the proximal section is advantageously utilized in the design of a delivery mechanism, that can be used to deploy such a surgical valve, as fewer parts of different size have to be made available for such a delivery system thereby simplifying this.

It is preferred if an outer diameter of the frame is larger either side of the transition present between the middle section and the distal section. This further increases the stability of the surgical valve and hence its lifetime.

The stent advantageously further comprises a valve to aid and/or replace the existing deficient valve. This valve is preferably arranged in or adjacent to the proximal section, with the proximal section being configured to receive the valve.

In this connection it should be noted that the valve can advantageously comprise at least one and preferably a plurality of leaflets and in particular two to five leaflets. The number of leaflets employed is selected in dependence on the location of deployment of the valve.

Advantageously, the outer diameter of the middle section increases at a transition from the proximal section to the middle section. This, on the one hand, clearly defines the transition from the proximal to the middle section. On the other hand, the middle section that is provided to anchor the surgical valve to either the IVC or the SVC does not interfere with the function or the positioning of the actual valve provided in the proximal section of the surgical valve.

It is preferred if the middle section has an outer shape that is selected from the group comprising part spherical, part oval shaped, part conically shaped, part truncated cone shaped and part frustoconical shaped. Such shapes permit an interference fit of the stent to the IVC or SVC without creating sharp points of contact between the IVC or SVC that could lead to a puncture or rupture of the IVC or SVC respectively.

Advantageously a length of the distal section corresponds to 35 to 75 % of the length of the middle section in the deployed state; and/or wherein the length of the distal section is equal to or shorter than the length of the proximal section. The provision of such a distal section enables a correct placement of the surgical valve using a specifically designed delivery system. This is because the deployment of the valve, using e.g. a transcatheter delivery system, can be brought about by holding the surgical valve at the location of deployment via the distal end prior to the expansion of the valve taking place at the intended location of deployment.

Advantageously the stent further comprises at least one piece of cuff material that is provided on an outer or inner surface of the frame. The provision of a cuff material, on the one hand, increases the lifetime of a surgical valve and, on the other hand, further reduces any risk of damaging the tissue in the region of deployment of the surgical valve. Moreover, the provision of a cuff material at at least some of the sections of the surgical valve also helps prevent leakage through the stent frame.

Advantageously the at least one piece of cuff material is made from a material selected from the group comprising biological material, natural material, artificial material and polymeric material. Such materials enable the creation of a surgical valve with an increased lifetime and with the necessary expandability.

Preferably at least two pieces of cuff material made from different material are provided. In this way e.g. different sections of the surgical valve can have different material properties that are tailored to the specific use of that section of the surgical valve.

Preferably a ratio of the lengths of the proximal section to the middle section to the distal section is selected from the range comprising at least substantially 1:2:1 to 4:8:3. These ratios enable a well-functioning valve to be deployed at the intended location with the required interference fit.

According to the invention the maximum outer diameter of the middle section corresponds to 130 to 170%, in particular to 140 to 160 % and most especially to at least substantially 150%, of the maximum outer diameter of the proximal section in the deployed state. This creates sufficient space at the location of deployment to ensure a correct functioning of the valve in addition to the correct adhering of the surgical valve to either one of the SVC or IVC. Moreover, selecting a middle section with such a large diameter ensures that the middle section attaches completely to the interior of the blood vessel. The middle section is thus intended to prevent paravalvular leakage and enhance anchoring of the implant to the anatomy of the blood vessel. Paravalvular leakage is defined as leakage around the side the prosthesis. This commonly occurs in valvular positions when the prosthesis does not properly appose the lumen wall.

The present disclosure also exemplifies a method of delivering and deploying a surgical valve comprising a stent in accordance with the invention. stent, the method not formming part of the invention.

The method comprises the steps of:
- inserting the surgical valve into one of the caval veins;
- placing the surgical valve at an atrial junction of the caval veins;
- expanding the surgical valve in the caval vein;
- attaching the surgical valve to the caval vein by means of at least one of an interference fit, sutures, staples, adhesively bonding, clamps and clips; and
- anchoring the distal section of the stent to the caval vein by means of the outwardly directed arms.

By means of this method the surgical valve can advantageously be deployed using minimally invasive techniques at the correct location of deployment.

The advantages described in connection with the surgical valve correspond to those or are similar to those that can be obtained by means of the method, wich does not form part of the invention.

The invention will be described in detail by means of embodiments and with reference to the drawings. These show preferred deployment locations and embodiments. The features described may be configured in various combinations, which are encompassed in this document. The drawings show:
- Fig. 1: an isometric view of a bare stent of a surgical valve;
- Fig. 2: a view of a surgical valve having a valve with three leaflets installed within the stent;
- Fig. 3: a further view of the coapted valve of Fig. 2;
- Fig. 4: the deployment of two surgical valves one in the SVC and one in the IVC; and
- Fig. 5: a valve deployed in the SVC.

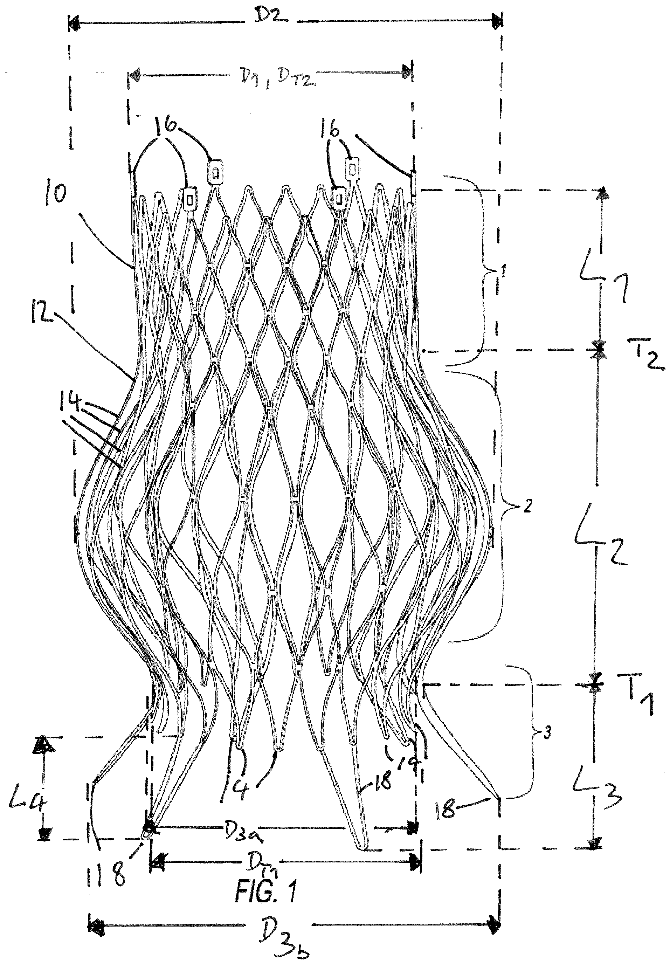

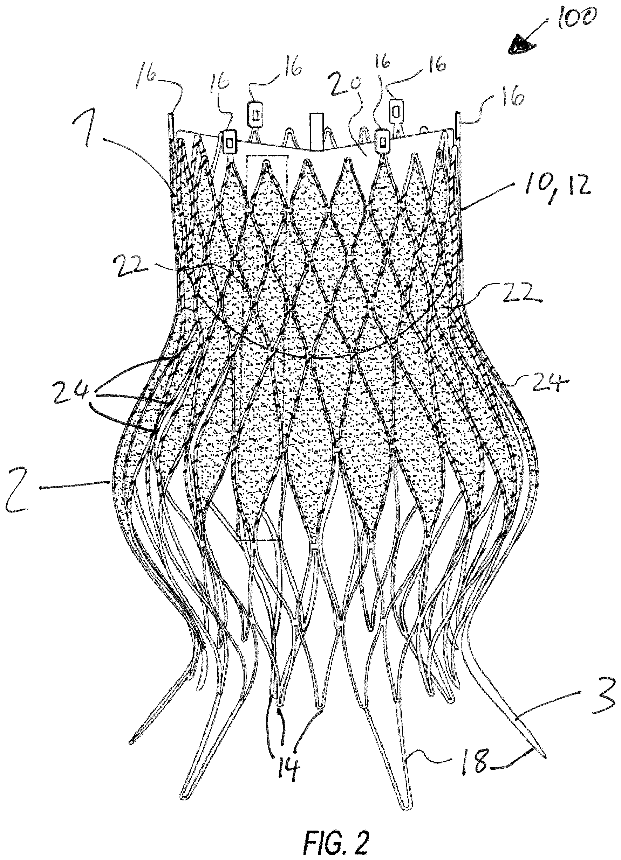

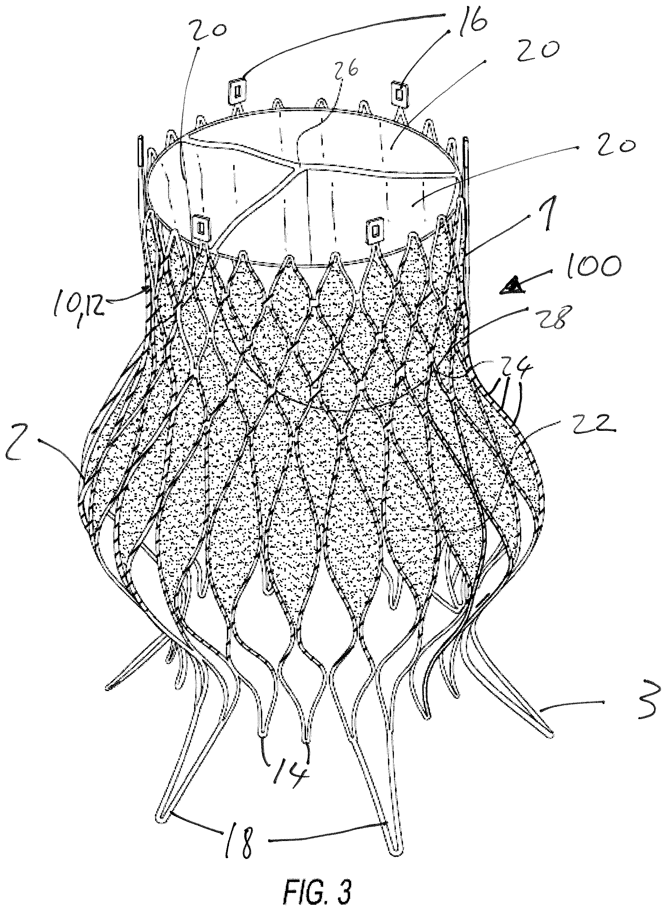

Fig.1 depicts an isometric view of a bare stent 10 of a surgical valve 100 without a cuff or leaflets (see Figs. 2 and 3) attached. The stent 10 has three distinct sections a proximal section 1, a middle section 2 and a distal section 3 forming a stent frame 12 that is composed of a plurality of interconnected first arms 14. The proximal section 1 further includes attachment features 16, such as tabs, to attach the surgical valve 100 to a delivery system (not shown).

It should be noted in this connection that when reference is made to the term proximal in this application, then that part of the surgical valve 100 respectively of the stent 10 which is intended to be positioned closest to a heart is currently being referred to. Consequently the term distal refers to that part of the surgical valve 100 respectively of the stent 10 that is to be disposed remote from the heart.

It should further be noted that the stent 10 respectively the surgical valve 100 disclosed herein is to be positioned in one of the caval veins, so that a blood flowing through the stent 10 to the heart flows from the distal section 3 to the proximal section 1. This means that the distal section 3 is a blood inflow region and the proximal section 1 is a blood outflow region.

The various sections 1, 2, 3 of the stent frame 16 are designed to satisfy different requirements. Namely the proximal section 1 - also known as the attachment region is, on the one hand, attached to the delivery system via e.g. the attachment features 16 and, on the other hand, is the region to which leaflets of a valve (see Fig. 3) are attached. In this connection it should be noted that the proximal section 1 can be cylindrical or conical in shape.

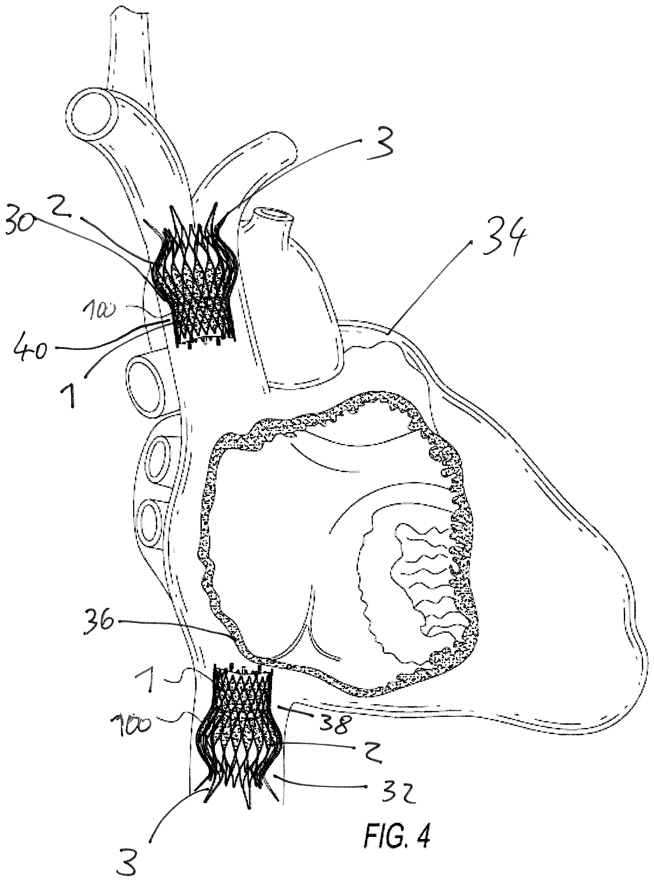

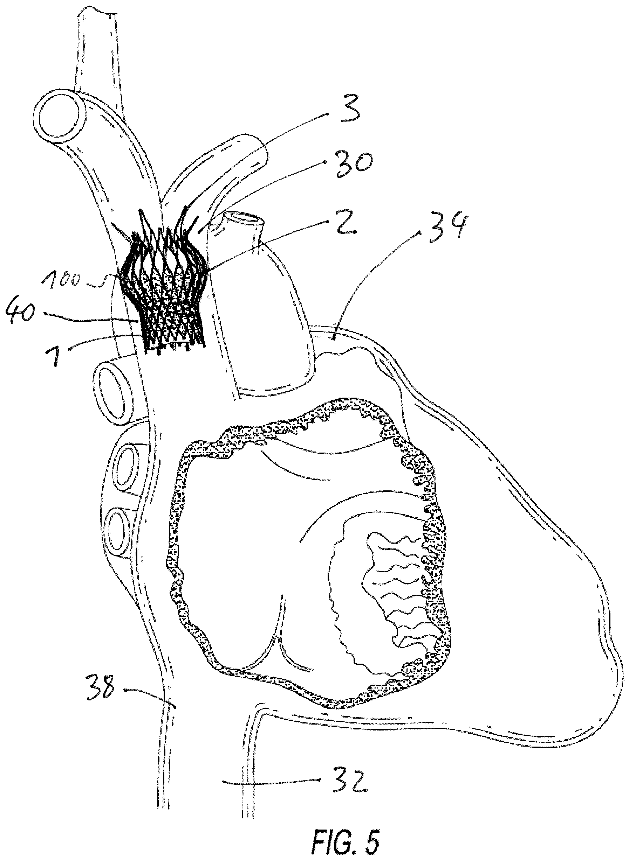

The middle section 2 is provided to prevent paravalvular leakage and assist with anchoring the surgical valve 100 to a vein (see Figs. 4 and 5). This section 2 has a greater diameter than that of the proximal section 1. In this specific instance a maximum outer diameter D₂ of the middle section 2 is approximately 150% of a maximum outer diameter D₁ of the proximal section 1. The stent 10 is designed with a large diameter of the middle section 2 to provide an interference fit with a vessel lumen (see Fig. 4 and 5).

The distal section 3 is designed to further anchor the surgical valve 100 within the anatomy of the vessel lumen. For this purpose a plurality of second arms 18 also known as anchoring arms are provided at a distal end.

The surgical valve 100 is designed for the heterotopic treatment of atrioventricular regurgitation that is expandable from an un-deployed state into a deployed state.

The Fig. 1 to 5 shown, all show the surgical valve 100 in the expanded state or deployed state. The following description of features generally relates to the deployed state of the surgical valve 100 unless something is stated to the contrary.

A length L₁ of the proximal section 1 corresponds to approximately 50% of a length L₂ of the middle section. Moreover, a length L₃ of the distal section 3 corresponds to approximately 50% of the length L₁ of the proximal section 1. Thus, a ratio of the lengths of the proximal section 1 to the middle section 2 to the distal section 3, i.e. L₁:L₂:L₃, is approximately 1:2:1.

The proximal section 1 has an at least substantially equal outer diameter D₁ over its length L₁. At a transition T₂ between the proximal section 1 and the middle section 2, the outer diameter of the middle section 2 increases starting from the proximal section 1 to the maximum outer diameter D₂ of the middle section 2. The middle section 2 has a part oval outer shape, such that starting from the maximum outer diameter D₂, the outer diameter of the middle section 2 decreases towards a transition T₁ located between the middle section 2 and the distal section 3.

The transition T₁ between the middle section 2 and the distal section 3 has an outer diameter D₃ₐ that is less than the maximum outer diameter D₂ of the middle section 2 and less than an outer diameter D_{3b} of the distal section 3. The diameter of the transition D₃ₐ at least substantially corresponds to the maximum outer diameter D₁ of the proximal section 1.

The plurality of second arms 18 that outwardly extend to the maximum outer diameter D_{3b} of the distal section 3, project beyond an end of the first arms 14 and extend over approximately 2/3 of the length L₃ of the distal section 3 beyond the end of the first arms 14. The maximum outer diameter D_{3b} of the distal section 3 at an outer end of the second arms 18 is approximately the same as the maximum outer diameter D₂ of the middle section 2.

This means that the first arms 14 have a length in the distal section 3 that corresponds to approximately 1/3 of the length L₃ of the distal section 3, whereas the second arms 18 have a length in the distal section that, due to their outwardly disposed nature, is longer than the length L₃ of the distal section 3.

The second arms 18 are approximately three to four times as long as the first arms 14 in the distal section 3 in the current embodiment. Generally speaking the first arms 14 can have a length that corresponds to approximately 20 to 70% of a length of the second arms 18 in the distal section 3.

The second arms 18 are elongated stent cells which extend radially from the stent 10. These arms can be evenly or irregularly spaced. Furthermore, the second arms 18 may remain uncovered or have a covering on top of the stent cells. The covering can include any kind of material that is deemed acceptable for use with cuffs or leaflets.

The radial force of the second arms 18 can be tailored to be strong to ensure adequate anchoring at the branch-off for the blood vessels in question. These anchoring arms 18 may be designed to further include active anchoring features, such as hooks, barbs or other similar features. It is also conceivable that the design of the arms is continuous around the stent 10 and has rounded edges to prevent vessel perforation.

An outer contour of the stent 10 of Fig. 1 can be described as having the following shapes, an approximately cylindrical shape in the proximal section 1, an approximately part spherical shape in the middle section 2, and a frustoconical shape in the distal section 3. This means that an outer contour of the stent 10 in a cross-section thereof is approximately linear in the proximal section 1, follows a part circle in the middle section 2 and then tapers outwardly again in the distal section 3.

In this connection it should be noted that the stent frame 12 is designed from a collapsible and flexible metal. The material can in this respect be superelastic, a shape memory alloy, self-expandable (e.g. Nitinol), ballon expandable (e.g. Cobalt chromium, Stainless Steel, etc) or polymeric or artificial. In order to manufacture the stent frame 12, this can be laser cut form a piece of tube (not shown), the stent frame 12 can be braided. Alternatively the different sections 1, 2, 3 of the stent frame 12 can be made of different materials or continuous material.

Additional anchoring of the stent 10 to a vein can be accomplished by means of sutures, barbs or hooks that can be further provided at the stent frame 12. Moreover, a radial force and in each section of the stent frame 12 can be adjusted via the materials selected for each section 1, 2, 3 of the stent frame 12, the specific (outer) dimensions selected for each section 1, 2, 3 or the processing of the specific sections 1, 2, 3 are dimensioned for location in the vena cava or pulmonary vein.

Fig. 2 shows the surgical valve 100 having a plurality of leaflets 20 installed therein. Moreover, the surgical valve 100 is provided with a cuff 22 that is attached to the plurality of first arms 14 of the stent frame 12 by means of a plurality of sutures 24. The cuff 22 extends from the proximal section 1 midway into the middle section 2.

The material of the cuff 22 may or may not be the same material as the material of the tissue, and may be an allograft, xenograft or a synthetic material. For example, the cuff 22 can be composed of biological, natural, artificial or polymeric materials or of cultured tissue.

The cuff 22 shown is attached to the inside of the stent 10. Naturally speaking it can also be attached at an outside of the stent 10.

The cuff 22 can be made of a single continuous piece as shown, or can be created from several pieces. If the cuff 22 is made from several pieces, the different pieces can be made from several different materials. The cuff 22 can be continuously attached to the stent 10 to cover the complete stent 10 or can be attached in different sections 1, 2, 3 or part sections 1, 2, 3 of the stent 10.

For example stents 10 can include short cuffs 22 which extend only throughout the proximal section 1.

More favorable embodiments, like that shown in Fig. 2, include a cuff 22, which extends at least midway into the middle section 2. This allows for the cuff 22 to seal against leakage both in the proximal section 1 and in the middle section 2.

In order to attach both the leaflets 20 and the cuff 20 to the stent 10 various attaching materials can be used. These include sutures 24, staples, glue, clamps, clips, etc.

The proximal section 1 is designed to support the plurality of leaflets 20 and the cuff 22. A correct mounting of the leaflets 20 and the cuff 22 enable a proper valve function. Leaflet 20 functionality is defined as the ability of the leaflets to properly coapt (close) and open during the desired flow cycles.

This region of the stent 10 can be either cylindrical or slightly conical or oval in geometry. A slightly conical shape indicates that each end of the cylinder of the proximal section 1 has different diameters, with one being greater than the other. If a conical shape of the proximal section 1 is selected then the larger diameter of the proximal section 1 may be designed to have a slight interference fit with the vessel it is implanted into.

The surgical valve 100 is secured or attached to the delivery system and/or within the delivery system (not shown) using a plurality of features on the stent, such as the tabs 16 shown in Fig. 2. These features may include passive or active mechanisms to attach to corresponding mating features in the delivery system. It is conceivable that either mechanism uses electrical, mechanical or magnetic components for securement of the prosthesis within the delivery system. It is also conceivable that the prosthesis is secured to the delivery system at either ends of the stent 10 - proximal or distal to the atrioventricular junction.

In this embodiment, the surgical valve 100 is intended to function as a heterotropic atrioventricular valve 26. The surgical valve 100 is intended to function as either a self-expandable or a balloon expandable valve for transcatheter procedures. For surgical procedures, it is conceivable that the prosthesis is implanted as is without using a delivery system.

Fig. 3 shows a surgical valve 100 with its leaflets 20 coapted, this means the valve 26 is in the closed state. In the present example the valve 26 comprises three leaflets 20. Fig. 3 shows the function of the valve 26 during backflow, this means when backflow from the heart should be prevented for which reason the valve is closed. In Fig. 3 a suture 28 is indicated, by means of which the leaflets 20 are attached in the proximal section 1.

The three leaflets 20 can be composed of biological, natural, artificial or polymeric materials or of cultured tissue or be made from xenograft cusps. In the example shown 3 leaflets are depicted.

Fig. 4 shows two surgical valves 100. One is deployed in the SVC 30 and the other is deployed in the IVC 32 of a human heart 34. In particular Fig. 4 shows the use of two prostheses 100 within the caval veins 30, 32 to depict dual valve implantation at one of the suggested atriocaval location for heterotropic treatment of tricuspid valve regurgitation. Furthermore, the lower surgical valve 100 implanted into the IVC 32 protrudes slightly into the right atrium 36, showing one possible deployment location.

The surgical valve 100 described herein is a valve prosthesis for the heterotopic treatment of tricuspid valve regurgitation at the location of the atrial junction within the inferior vena cava (IVC) and the superior vena cava (SVC) respetively. The surgical valve 100 may be implanted using transcatheter techniques through incisions in femoral veins, jugular veins, or similar locations to access the desired implantation site.

It is also conceivable that such a surgical valve 100 may be implanted through surgical intervention.

For a transcatheter placement of the surgical valve 100, a delivery system may be used to transport the implant through the vasculature to the desired implantation - region. It is conceivable that the same delivery system will either deploy a single prosthesis 100 or a total of two total prostheses. If a dual valve delivery system is used, the order of deployment will be determined by the location of approach of the delivery system.

The desired landing zone for the valvular apparatus of the surgical valve 100 at the caval location includes protrusion of the valvular apparatus into the right atrium 36, located at the right atrial annulus 38 and located within the superior and/or inferior vena cava cranial 40 and caudal (respectively) to the atrial annular opening. It is reasonable that the overall geometry of the stent 10 (height, diameter, dimensions of each region) is tailored to fit specific anatomical needs in patients.

The surgical valve 100 described may also be a surgical valve for the heterotopic treatment of atrioventricular regurgitation.

Fig. 5 depicts an embodiment where the surgical valve 100 is only deployed in the SVC to show a singular surgical valve 100 implantation at one of the atriocaval location within the lumen of the vein.

In a different embodiment of the method, it is conceivable that the device may be implanted through surgical methods. This includes direct implantation at the site of the IVC and SVC by surgical cut-down close to the thorax. It is also conceivable that the prosthesis is implanted at either site or both sites in a single procedure.

In another embodiment of the design, it is conceivable that the surgical valve 100 is used for the treatment of mitral valve regurgitation with orthotopic valve placement at the site of the pulmonary vein. It is also reasonable that the placement of the surgical valve 100 is achieved either by surgery or transcatheter options. It is also conceivable that for the transcatheter options, the access site may include but are not limited to the femoral artery, femoral vein, apical access, subclavian access (trans-aortic) or miscellaneous.

In another embodiment of the design, it is conceivable that the design is used as an atrioventricular valve. Specifically, it is conceivable that the device is used orthotopically at the site of the tricuspid valve annulus and at the mitral valve annulus for the treatment of either native valve regurgitation. It is also conceivable that in another embodiment the same design is used for the treatment of diseases affecting the aortic valve and the pulmonary valve.

### List of reference numerals:

- 1: proximal section
- 2: middle section
- 3: distal section
- 10: stent
- 12: stent frame
- 14: first arms
- 16: attachment features
- 18: second arms
- 20: leaflet
- 22: cuff
- 24: suture
- 26: valve
- 28: suture
- 30: superior vena cava (SVC)
- 32: inferior vena cava (IVC)
- 34: heart
- 36: right atrium
- 38: right atrial annulus
- 40: vena cava cranial
- 100: surgical valve

- D₁: diameter
- D₂: diameter
- D₃ₐ: diameter
- D_{3b}: diameter
- D_{T1}: diameter
- D_{T2}: diameter
- L₁: length
- L₂: length
- L₃: length
- T₁: transition
- T₂: transition

## Claims

1. A stent (10) for a surgical valve (100) for the heterotopic treatment of atrioventricular regurgitation that is expandable from an un-deployed state into a deployed state, and that comprises a frame (12) composed of a plurality of first arms (14) forming three interconnected sections, a proximal section (1), a distal section (3) and a middle section (2) disposed between the proximal section (1) and the distal section (3), wherein, in the deployed state, a maximum outer diameter of the middle section (2) is larger than a maximum outer diameter of the proximal section (1) and a length of the proximal section (1) corresponds to 35 to 70% of a length of the middle section (2),
wherein the outer diameter of the middle section (2) increases at a transition from the proximal section (1) to the middle section (2), and
wherein, in the deployed state, the maximum outer diameter of the middle section (2) corresponds to 130 to 170% of the maximum outer diameter of the proximal section (1)
wherein the proximal section (1) has an at least substantially equal outer diameter (D₁) over its length, wherein, at a transition (T₂) between the proximal section (1) and the middle section (2), the outer diameter of the middle section (2) increases starting from the proximal section (1) to the maximum outer diameter (D₂) of the middle section (2), wherein, starting from the maximum outer diameter (D₂,) the outer diameter of the middle section (2) decreases towards a transition (T₁) located between the middle section (2) and the distal section (3) and wherein, in the deployed state, the plurality of first arms (14) is outwardly directed in the distal section (3) of the frame (12).

2. A stent (10) in accordance with claim 1,
wherein the distal section (3) further comprises a plurality of second arms (18) that outwardly extend to a maximum outer diameter of the distal section (3), wherein the plurality of second arms (18) extends beyond a distal end of the first arms (14).

3. A stent (10) in accordance with claim 1 or claim 2,
wherein a maximum outer diameter of the distal section (3) is at least approximately the same as a maximum outer diameter of the middle section (2) in the deployed state.

4. A stent (10) in accordance with at least one of the preceding claims, wherein a transition between the middle section (2) and the distal section (3) has an outer diameter that is less than the maximum outer diameter of the middle section (2) and less than an outer diameter of the distal section (3) in the deployed state.

5. A stent (10) in accordance with claim 4,
wherein, in the deployed state, a diameter of the transition at least substantially corresponds to the maximum outer diameter of the proximal section (1) or is less than the maximum outer diameter of the proximal section (1).

6. A stent (10) in accordance with claim 4 or claim 5,
wherein an outer diameter of the frame (12) is larger either side of the transition between the middle section (2) and the distal section (3).

7. A stent (10) in accordance with at least one of the preceding claims,
wherein the middle section (2) has an outer shape that is selected from the group comprising part spherical, part oval shaped, part conically shaped, part truncated cone shaped and part frustoconical shaped.

8. A stent (10) in accordance with at least one of the preceding claims,
wherein a length of the distal section (3) corresponds to 35 to 75 % of the length of the middle section (2) in the deployed state.

9. A stent (10) in accordance with at least one of the preceding claims,
wherein the length of the distal section (3) is equal to or shorter than the length of the proximal section (1).

10. A stent (10) in accordance with at least one of the preceding claims, further comprising at least one piece of cuff material that is provided on an outer or inner surface of the frame (12).

11. A stent (10) in accordance with claim 10,
wherein the at least one piece of cuff material is made from a material selected from the group comprising biological material, natural material, artificial material and polymeric material.

12. A stent (10) in accordance with claim 10,
wherein at least two pieces of cuff material made from different material are provided.

13. A stent frame (10) in accordance with at least one of the preceding claims, further comprising a valve; and wherein the proximal section (1) is optionally configured to receive the valve.

14. A stent (10) in accordance with at least one of the preceding claims, wherein a ratio of the lengths of the proximal section (1) to the middle section (2) to the distal section (3) is selected from the range comprising at least substantially 1:2:1 to 4:8:3.

## Patentansprüche

1. Stent (10) für eine chirurgische Klappe (100) zur heterotopen Behandlung von atrioventrikulärer Regurgitation, der von einem nicht entfalteten Zustand in einen entfalteten Zustand expandierbar ist und der einen Rahmen (12) umfasst, der aus einer Vielzahl von ersten Armen (14) besteht, die drei miteinander verbundene Abschnitte bilden, einen proximalen Abschnitt (1), einen distalen Abschnitt (3) und einen mittleren Abschnitt (2), der zwischen dem proximalen Abschnitt (1) und dem distalen Abschnitt (3) angeordnet ist), wobei im entfalteten Zustand ein maximaler Außendurchmesser des mittleren Abschnitts (2) größer ist als ein maximaler Außendurchmesser des proximalen Abschnitts (1) und eine Länge des proximalen Abschnitts (1) 35 bis 70% einer Länge des mittleren Abschnitts (2) entspricht, wobei der Außendurchmesser des mittleren Abschnitts (2) an einem Übergang von dem proximalen Abschnitt (1) zu dem mittleren Abschnitt (2) zunimmt, und
wobei im entfalteten Zustand der maximale Außendurchmesser des mittleren Abschnitts (2) 130 bis 170 % des maximalen Außendurchmessers des proximalen Abschnitts (1) entspricht),
wobei der proximale Abschnitt (1) über seine Länge einen zumindest im Wesentlichen gleichen Außendurchmesser (D₁) aufweist, wobei an einem Übergang (T₂) zwischen dem proximalen Abschnitt (1) und dem mittleren Abschnitt (2) der Außendurchmesser des mittleren Abschnitts (2) ausgehend von dem proximalen Abschnitt (1) bis zu dem maximalen Außendurchmesser (D₂) des mittleren Abschnitts (2) zunimmt, wobei ausgehend von dem maximalen Außendurchmesser (D₂) der Außendurchmesser des mittleren Abschnitts (2) in Richtung eines Übergangs (T₁) abnimmt, der zwischen dem mittleren Abschnitt (2) und dem distalen Abschnitt (3) angeordnet ist, und wobei in dem entfalteten Zustand die Mehrzahl von ersten Armen (14) in dem distalen Abschnitt (3) des Rahmens (12) nach außen gerichtet ist.

2. Stent (10) nach Anspruch 1,
wobei der distale Abschnitt (3) ferner eine Vielzahl von zweiten Armen (18) umfasst, die sich nach außen bis zu einem maximalen Außendurchmesser des distalen Abschnitts (3) erstrecken, wobei sich die Vielzahl der zweiten Arme (18) über ein distales Ende der ersten Arme (14) hinaus erstreckt.

3. Stent (10) nach einem der Ansprüche 1 oder 2,
wobei ein maximaler Außendurchmesser des distalen Abschnitts (3) zumindest annähernd der gleiche ist wie ein maximaler Außendurchmesser des mittleren Abschnitts (2) im entfalteten Zustand.

4. Stent (10) nach einem der vorhergehenden Ansprüche,
wobei ein Übergang zwischen dem mittleren Abschnitt (2) und dem distalen Abschnitt (3) einen Außendurchmesser hat, der kleiner als der maximale Außendurchmesser des mittleren Abschnitts (2) und kleiner als ein Außendurchmesser des distalen Abschnitts (3) im entfalteten Zustand ist.

5. Stent (10) nach Anspruch 4,
wobei im entfalteten Zustand ein Durchmesser des Übergangs zumindest im Wesentlichen dem maximalen Außendurchmesser des proximalen Abschnitts (1) entspricht oder kleiner als der maximale Außendurchmesser des proximalen Abschnitts (1) ist.

6. Stent (10) nach einem der Ansprüche 4 oder 5,
wobei ein Außendurchmesser des Rahmens (12) auf beiden Seiten des Übergangs zwischen dem mittleren Abschnitt (2) und dem distalen Abschnitt (3) größer ist.

7. Stent (10) nach einem der vorhergehenden Ansprüche,
wobei der mittlere Abschnitt (2) eine äußere Form hat, die aus der Gruppe ausgewählt ist, die teilweise kugelförmig, teilweise oval, teilweise kegelförmig, teilweise als abgeflachter Kegel geformt und teilweise kegelstumpfförmig ist.

8. Stent (10) nach einem der vorhergehenden Ansprüche,
wobei eine Länge des distalen Abschnitts (3) 35 bis 75 % der Länge des mittleren Abschnitts (2) im entfalteten Zustand entspricht.

9. Stent (10) nach einem der vorhergehenden Ansprüche,
wobei die Länge des distalen Abschnitts (3) gleich oder kürzer als die Länge des proximalen Abschnitts (1) ist.

10. Stent (10) nach einem der vorhergehenden Ansprüche,
ferner mit mindestens einem Stück aus Manschettenmaterial, das an einer Außen- oder Innenfläche des Rahmens (12) angebracht ist.

11. Stent (10) nach Anspruch 10,
wobei das mindestens eine Stück aus Manschettenmaterial aus einem Material hergestellt ist, das aus der Gruppe ausgewählt ist, die biologisches Material, natürliches Material, künstliches Material und Polymermaterial umfasst.

12. Stent (10) nach Anspruch 10,
wobei mindestens zwei Stücke aus Manschettenmaterial, die aus unterschiedlichem Material hergestellt sind, vorgesehen sind.

13. Stent (10) nach einem der vorhergehenden Ansprüche,
ferner mit einer Klappe, wobei der proximale Abschnitt (1) optional zur Aufnahme der Klappe konfiguriert ist.

14. Stent (10) nach einem der vorhergehenden Ansprüche,
wobei ein Verhältnis der Längen des proximalen Abschnitts (1) zum mittleren Abschnitt (2) zum distalen Abschnitt (3) aus dem Bereich ausgewählt ist, der mindestens im Wesentlichen 1:2:1 bis 4:8:3 umfasst.

## Revendications

1. Stent (10) destiné à une valvule chirurgicale (100) pour le traitement hétérotopique d'une régurgitation atrio-ventriculaire qui peut s'étendre depuis un état non déployé jusque dans un état déployé, et qui comprend un cadre (12) composé d'une pluralité de premiers bras (14) formant trois sections interconnectées, une section proximale (1), une section distale (3) et une section médiane (2) disposé entre la section proximale (1) et la section distale (3), dans lequel, dans l'état déployé, un diamètre extérieur maximum de la section médiane (2) est plus grand qu'un diamètre extérieur maximum de la section proximale (1) et une longueur de la section proximale (1) correspond à 35 à 70 % d'une longueur de la section médiane (2), dans lequel le diamètre extérieur de la section médiane (2) augmente au niveau d'une transition depuis la section proximale (1) jusqu'à la section médiane (2), et dans lequel, dans l'état déployé, le diamètre extérieur maximum de la section médiane (2) correspond à 130 à 170 % du diamètre extérieur maximum de la section proximale (1),
dans lequel la section proximale (1) a un diamètre extérieur (D₁) au moins sensiblement égal sur sa longueur, dans lequel, au niveau d'une transition (T₂) entre la section proximale (1) et la section médiane (2), le diamètre extérieur de la section médiane (2) augmente en démarrant depuis la section proximale (1) jusqu'au diamètre extérieur maximum (D₂) de la section médiane (2), dans lequel, en démarrant depuis le diamètre extérieur maximum (D₂), le diamètre extérieur de la section médiane (2) diminue vers une transition (T₁) située entre la section médiane (2) et la section distale (3), et dans lequel, dans l'état déployé, la pluralité de premiers bras (14) sont dirigés vers l'extérieur dans la section distale (3) du cadre (12).

2. Stent (10) selon la revendication 1,
dans lequel la section distale (3) comprend en outre une pluralité de seconds bras (18) qui s'étendent vers l'extérieur jusqu'à un diamètre extérieur maximum de la section distale (3), dans lequel la pluralité de seconds bras (18) s'étende au-delà d'une extrémité distale des premiers bras (14).

3. Stent (10) selon la revendication 1 ou 2,
dans lequel un diamètre extérieur maximum de la section distale (3) est au moins approximativement le même qu'un diamètre extérieur maximum de la section médiane (2) dans l'état déployé.

4. Stent (10) selon l'une au moins des revendications précédentes,
dans lequel une transition entre la section médiane (2) et la section distale (3) a un diamètre extérieur qui est inférieur au diamètre extérieur maximum de la section médiane (2) et qui est inférieur à un diamètre extérieur de la section distale (3) dans l'état déployé.

5. Stent (10) selon la revendication 4,
dans lequel, dans l'état déployé, un diamètre de la transition correspond au moins sensiblement au diamètre extérieur maximum de la section proximale (1) ou est inférieur au diamètre extérieur maximum de la section proximale (1).

6. Stent (10) selon la revendication 4 ou 5,
dans lequel un diamètre extérieur du cadre (12) est plus grand de chaque côté de la transition entre la section médiane (2) et de la section distale (3).

7. Stent (10) selon l'une au moins des revendications précédentes,
dans lequel la section médiane (2) a une forme extérieure qui est sélectionnée parmi le groupe comprenant : une partie sphérique, une partie de forme ovale, une partie de forme conique, une partie en forme de cône tronqué et une partie tronconique.

8. Stent (10) selon l'une au moins des revendications précédentes,
dans lequel une longueur de la section distale (3) correspond à 35 à 75 % de la longueur de la section médiane (2) dans l'état déployé.

9. Stent (10) selon l'une au moins des revendications précédentes,
dans lequel la longueur de la section distale (3) est égale ou plus courte que la longueur de la section proximale (1).

10. Stent (10) selon l'une au moins des revendications précédentes,
comprenant en outre au moins une pièce de matière de manchette qui est prévue sur une surface extérieure ou intérieure du cadre (12).

11. Stent (10) selon la revendication 10,
dans lequel ladite au moins une pièce de matériau de manchette est faite à partir d'un matériau sélectionné parmi le groupe comprenant : une matière biologique, une matière naturelle, une matière artificielle et une matière polymère.

12. Stent (10) selon la revendication 10,
dans lequel il est prévu au moins deux pièces de matériaux de manchette faites de matières différentes.

13. Cadre de stent (10) selon l'une au moins des revendications précédentes,
comprenant en outre une valvule ; et dans lequel la section proximale (1) est configurée en option pour recevoir la valvule.

14. Stent (10) selon l'une au moins des revendications précédentes,
dans lequel un rapport entre les longueurs de la section proximale (1), de la section médiane (2) et de la section distale (3) est sélectionné dans une plage allant au moins sensiblement de 1:2:1 à 4:8:3.
